# EUROPÄISCHE PATENTSCHRIFT

(11) **EP 0 778 041 B1**
(45) Veröffentlichungstag und Bekanntmachung des Hinweises auf die Patenterteilung: **21.03.2001**
(21) Anmeldenummer: 96119587.2
(22) Anmeldetag: 06.12.1996
(51) Int. Cl.: A61M 25/02

(54) **Vorrichtung zur Stabilisierung der Haut eines Menschen oder Tieres**
Device for stabilizing the human or animal skin
Dispositif pour stabiliser la peau humaine ou de l'animal

(30) Priorität: 08.12.1995 DE 29519451 U
(43) Veröffentlichungstag der Anmeldung: 11.06.1997
(73) Patentinhaber: Aigner, Manfred, Dr. med., 85080 Gaimersheim (DE); Krause, Dieter, Dr. med., 85139 Lenting (DE)
(72) Erfinder: Aigner, Manfred, Dr. med., 85080 Gaimersheim (DE); Krause, Dieter, Dr. med., 85139 Lenting (DE)
(74) Vertreter: Sasse, Volker, Dipl.-Ing.

(56) Entgegenhaltungen:
- US-A- 5 364 368
- US-A- 5 380 294
- US-A- 5 468 231

## Beschreibung

Die Erfindung betrifft eine Vorrichtung zur Stabilisierung der Haut gemäß dem Oberbegriff des Patentanspruchs 1.

Aus der Praxis sind bereits Heftpflaster bekannt, mit denen in die Haut eingebrachte Injektionsnadeln festgelegt sind. Diese Heftpflaster bestehen aus einer biege- und zugelastischen Trägerschicht, die einseitig mit Klebstoff beschichtet ist, so daß sie gut auf der Haut haftet. Wird diese Vorrichtung an der Austrittsstelle eines Katheters angebracht, indem sie über die Haut und den Katheter geklebt wird, so wird dieser durch diese Vorrichtung gehalten. Aufgrund der Elastizität dieser Vorrichtung kann diese jedoch die Haut und die darunterliegenden Gewebeschichten nicht stabilisieren, so daß diese gegenüber dem Katheter bewegt werden kann. Dies führt jedoch zu unerwünschten Pressungen des Gewebes am Katheter, so daß die Zellen in der Umgebung des Katheters geschädigt werden und absterben. Die so geschädigten Zellschichten bilden jedoch für eindringende Bakterien einen idealen Nährboden, was zu einem erhöhten Infektionsrisiko führt. Dies ist insbesondere bei Kathetern problematisch, die durch die Bauchwand durchgeführt sind, da diese bei Bewegungen der Person und insbesondere bei der Atmung ständig bewegt wird. Das Infektionsrisiko wird durch die Affinität bestimmter Bakterien, insbesondere Staphylokokken, zu Kunststoffen, aus denen die Katheter bestehen, weiter erhöht.

Aus der WO 95/20415 ist eine Vorrichtung zur Stabilisierung der Haut in der Umgebung der Austrittsstelle eines Katheters bekannt. Diese Vorrichtung besteht aus einer dünnen, biegeelastischen Trägerschicht, die eine die Austrittsstelle teilweise umfassende Aussparung aufweist. Diese Trägerschicht ist unterseitig mit einer Klebeschicht belegt, um die Vorrichtung an der Haut anbringen zu können. Oberseitig ist an der Trägerschicht eine Schutzschicht angebracht, die ausschließlich für die Handhabung gedacht ist und der Trägerschicht während des Aufklebens auf die Haut eine erhöhte Steifigkeit verleiht. Nach dem Aufkleben der Trägerschicht wird diese Schutzschicht entfernt.

Der Erfindung liegt daher die Aufgabe zugrunde, eine Vorrichtung der eingangs genannten Art zu schaffen, bei der die Haut soweit stabilisiert wird, daß eine Traumatisierung der Haut im Katheterbereich auch bei Bewegungen der Person weitgehend reduziert ist.

Diese Aufgabe wird erfindungsgemäß mit den Merkmalen des Anspruchs 1 gelöst.

Die Trägerschicht ist biegeelastisch und vorzugsweise auch zugelastisch ausgebildet, so daß sie sich ausreichend gut an die Oberflächenkrümmung der Haut anpaßt. Da die Trägerschicht mit einem zugfesten Material verstärkt ist, wird die Haut im Bereich der Austrittsstelle des Katheters soweit ruhiggestellt, daß Zellschädigungen im Bereich des Katheters weitgehend reduziert sind. Bei einer Bewegung der Haut, beispielsweise durch Anspannen darunterliegender Muskeln, wird diese in der Nähe der Katheter-Austrittsstelle nicht mehr gegen den Katheter gedrückt. Es ist jedoch wichtig, daß die Trägerschicht trotz der Verstärkung biegsam bleibt, damit sie sich an die jeweilige Oberflächenkrümmung der Haut anpaßt. Dabei kann das die Trägerschicht verstärkende Material an der der Klebeschicht gegenüberliegenden Seite vorgesehen sein. Vorzugsweise wird das zugfeste Material mit der Trägerschicht verklebt oder verschweißt, so daß von der Haut auf die Trägerschicht übertragene Zugkräfte vom zugfesten Material abgefangen werden. Eine Verschweißung ist insbesondere dann vorteilhaft, wenn sowohl die Trägerschicht als auch das zugfeste Material aus Kunststoff gefertigt sind. Besteht die Trägerschicht dagegen aus einem textilartigen Material, was insbesondere für die gewünschte Luftdurchlässigkeit der Vorrichtung vorteilhaft ist, so könnte das zugfeste Material mit der Trägerschicht verwoben werden. Auch dadurch ist eine günstige Zugkraftübertragung von der Haut auf das zugfeste Material gewährleistet. Außerdem sind hierzu keine zusätzlichen Chemikalien zum Kleben oder Schweißen des zugfesten Materials erforderlich, die ggf. zu Hautreizungen führen könnten. Dies ist insbesondere deshalb von Bedeutung, da der Katheter je nach Anwendungsfall teilweise über Jahre im Körper verbleibt, so daß sich auch schwach reizende Stoffe sehr negativ auf die Katheter-Austrittsstelle auswirken. Wichtig ist auch, daß die Aussparung der Vorrichtung die Austrittsstelle des Katheters umfaßt, damit die Haut in der Nähe der Katheter-Austrittsstelle gut stabilisiert ist. Der Katheter durchdringt die Vorrichtung im Bereich der Aussparung. Die Aussparung umfaßt die Katheter-Austrittsstelle vorzugsweise eng, so daß der Abstand des zugfesten Materials vom Katheter geringer als der Katheterdurchmesser ist. Besonders günstig ist es, wenn das zugfeste Material der Trägerschicht bis an die Aussparung heranreicht, um die Haut auch direkt neben dem Katheter hinreichend zu stabilisieren. Insbesondere bei Kathetern, die durch die Bauchdecke geführt sind, ergibt sich das Problem, daß die Haut beim Sitzen und Vorbeugen in Falten gelegt wird. Dies verursacht erhebliche Schubkräfte, so daß auch die Vorrichtung zur Faltenbildung neigt. Diese wird durch die stauchfeste Ausbildung der Vorrichtung unterbunden, so daß auch unter diesen Bedingungen eine sichere Stabilisierung der Haut gewährleistet ist. Da der Katheter ggf. über Jahre hinweg getragen werden muß, ist es besonders wichtig, daß auch das zugfeste Material luftdurchlässig ist, damit die Hautatmung und Feuchtigkeitsabgabe ermöglicht ist.

Besteht die Trägerschicht gemäß Anspruch 2 selbst aus dem zugfesten Material, so läßt sich die Vorrichtung besonders einfach und damit kostengünstig herstellen. Ein Verbinden der Trägerschicht mit dem zugfesten Material entfällt, so daß auch der Einsatz von hierfür erforderlichen Chemikalien vermieden wird. Hierdurch reduziert sich die Gefahr von Hautreizungen, die insbesondere durch Allergien hervorgerufen werden.

Die gewünschte Luftdurchlässigkeit der Vorrichtung läßt sich gemäß Anspruch 3 sehr einfach dadurch gewährleisten, daß das zugfeste Material gitterartig aufgebaut ist. Zwischen den einzelnen Fäden, Streifen bzw. Bändern, die das Gitter beispielsweise im Flechtwerk bilden, kann Luft bzw. Feuchtigkeit ausgetauscht werden, so daß Hautschädigungen aufgrund eines Luftabschlusses verhindert werden.

Alternativ könnte das zugfeste Material auch eine mit Durchbrüchen versehene Folie sein. Eine Folie besitzt eine besonders hohe Zugfestigkeit, die durch die Durchbrüche nur wenig reduziert wird, wobei die Durchbrüche einen hinreichenden Luftaustausch erlauben, so daß Hautschädigungen vermieden werden. Außerdem läßt eine Folie zwar Biegungen jedoch kein Stauchen zu.

Gemäß Anspruch 4 hat sich für das zugfeste Material der Einsatz von Kunststoff bewährt. Kunststoffe lassen sich einfach und damit preisgünstig in verschiedenen Formen herstellen und bieten im Verhältnis zu ihrem Gewicht eine hohe Zugfestigkeit. Sie sind trotzdem sehr leicht biegsam, so daß sie sich der jeweiligen Oberflächenkrümmung der Haut anpassen.

Besonders günstig ist es, wenn gemäß Anspruch 5 das zugfeste Material mit Fasern armiert ist. Dabei ist unter anderem an Kohle-, Glas- oder ähnliche Fasern gedacht. Diese Fasern, die im wesentlichen parallel zur Trägerschicht liegen, besitzen eine sehr hohe Zugfestigkeit, so daß die Haut besonders günstig stabilisiert wird. Da die Fasern quer zu ihrer Längserstreckung keine Kraft aufnehmen können, läßt sich dieses Material trotzdem sehr leicht biegen, so daß es sich gut der jeweiligen Hautoberfläche anpaßt.

Gemäß Anspruch 6 ist es günstig, wenn die verstärkt ausgebildete Trägerschicht außenseitig mit einem zugelastischen Randstreifen umgeben ist. Dieser Randstreifen sorgt für eine gute, flächige Verbindung der Vorrichtung mit der Haut. Dadurch wird vermieden, daß sich die Vorrichtung bei einer Bewegung der Haut teilweise ablöst, womit die Stabilisierungswirkung verlorenginge. Für den Randstreifen hat sich je nach Größe der Vorrichtung eine Breite von 1 bis 3 cm bewährt.

Gemäß Anspruch 7 ist es vorteilhaft, die Aussparung als eine in der Trägerschicht vorgesehene Durchbrechung auszubilden. Hierdurch wird allseitig um die Katheter-Austrittsstelle eine Stabilisierung der Haut erreicht. Vorzugsweise ist die Durchbrechung mittig zur Trägerschicht angeordnet. Dies bewirkt eine besonders gleichmäßige, allseitige Stabilisierung der Haut um die Katheter-Austrittsstelle. Um die Vorrichtung einfach auf der Haut anbringen und von ihr wieder entfernen zu können, ist es vorteilhaft, einen Schnitt von ihrem äußeren Rand bis zur Durchbrechung vorzusehen. Damit läßt sich die Vorrichtung durch einfaches Auseinanderziehen der Bereiche beidseits des Schnittes über den Katheter schieben, bis dieser die Vorrichtung an ihrer Durchbrechung durchsetzt. Alternativ kann die Aussparung auch am Rand der Trägerschicht vorgesehen werden. In diesem Fall kann die Vorrichtung die Haut zwar nur von einer Seite stabilisieren, dies kann jedoch sehr einfach durch Anbringen zweier Vorrichtungen ausgeglichen werden, die zusammen den Katheter umfassen. Insbesondere ist das Anbringen der Vorrichtung besonders einfach, da sie lediglich mit ihrer Aussparung dicht an den Katheter herangeführt und anschließend auf die Haut aufgeklebt werden muß.

Gemäß Anspruch 8 ist es vorteilhaft, die verstärkte Trägerschicht am Rand der Aussparung mit einer Wulst zu versehen. Diese Wulst sorgt für eine zusätzliche Stabilisierung der Vorrichtung rund um die Katheter-Austrittsstelle. Auf diese Weise werden Pressungen bzw. Faltenbildungen der Haut in unmittelbarer Nähe der Katheter-Austrittsstelle zuverlässig vermieden. Umfaßt die Wulst den Katheter um mehr als 180°, so fixiert sie den Katheter ähnlich einem Clipverschluß. Vorzugsweise umfaßt die Wulst den Katheter um nur wenig mehr als 180°, so daß der Katheter leicht in die Wulst eingepaßt werden kann. Die Wulst verhindert eine Bewegung des Katheters unterhalb der Katheter-Austrittsstelle, die beispielsweise durch Reiben des aus der Haut herausragenden Teils an der Kleidung der Person verursacht werden könnte. Dies ist wichtig, da auch eine Bewegung des Katheters eine Traumatisierung der Haut verursachen kann.

Zusätzlich oder alternativ kann gemäß Anspruch 9 am zugfesten Material mindestens ein Halteclip für den Katheter vorgesehen sein. Dieser Halteclip kann den Katheter in unmittelbarer Nähe seiner Austrittsstelle fixieren. Andererseits wäre es auch denkbar, den Katheter auf der Vorrichtung aufzuwickeln und sein Ende im Halteglied zu fixieren. Dies ist insbesondere dann vorteilhaft, wenn der Patient duschen will, wobei der Katheter stören würde. Selbstverständlich können auch mehrere Halteclips vorgesehen sein, die den Katheter unmittelbar an seiner Austrittsstelle und an seinem Gegenende fixieren.

Schließlich ist es gemäß Anspruch 10 günstig, die Aussparung zusammen mit dem Katheter vorzugsweise mit einem Pflaster abzudecken. Dies verhindert ein Eindringen von Schmutz und Fäden der Kleidung in die Katheter-Austrittsstelle. Damit kann die Katheter-Austrittsstelle leichter steril gehalten werden, was das Infektionsrisiko weiter herabsetzt.

Anhand der Zeichnung werden bevorzugte Ausführungsformen des Erfindungsgegenstandes beispielhaft erläutert, ohne den Schutzumfang zu beschränken.

Es zeigt:
- Figur 1: eine Draufsicht auf eine Vorrichtung zur Stabilsierung der Haut mit eingelegtem Katheter,
- Figur 2: eine Schnittdarstellung durch die Vorrichtung gemäß Figur 1 entlang der Schnittlinie II-II,
- Figur 3: eine Schnittdarstellung durch die Vorrichtung gemäß Figur 1 entlang der Schnittlinie III-III,
- Figur 4: eine vergrößerte Schnittdarstellung der Vorrichtung gemäß Figur 2,
- Figur 5: eine vergrößerte Schnittdarstellung einer weiteren Ausführungsform der Vorrichtung und
- Figur 6: eine Draufsicht auf eine Vorrichtung zur Stabilisierung der Haut mit zwei Halteclips.

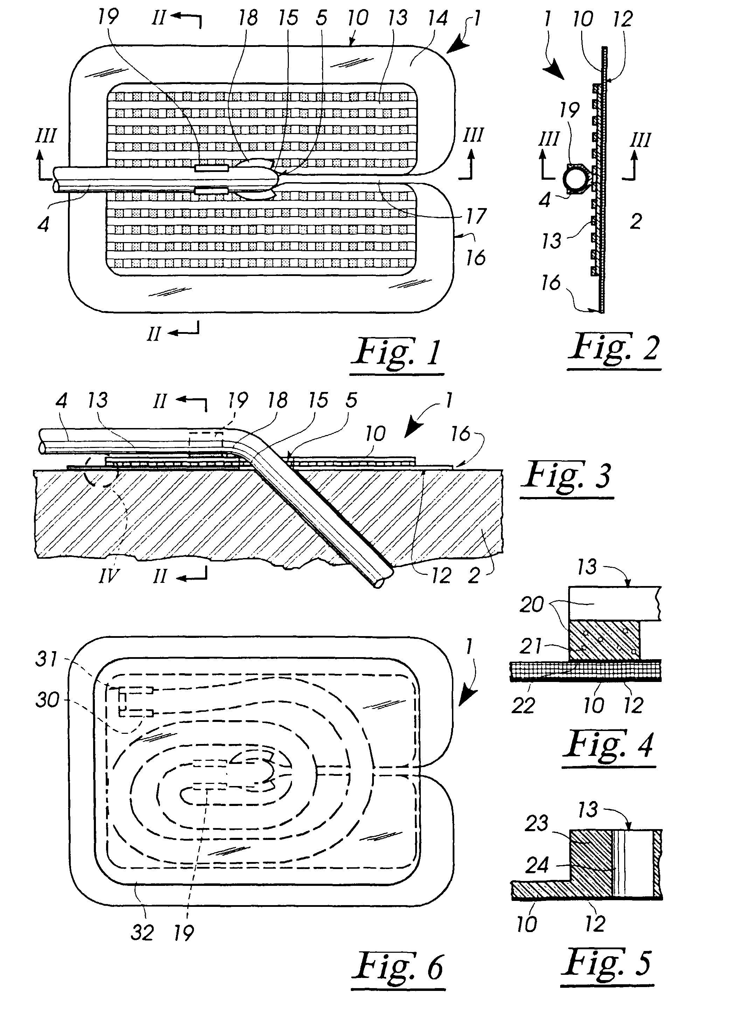

Die Figuren 1 bis 3 zeigen verschiedene Darstellungen einer Vorrichtung 1 zur Stabilisierung einer Haut 2. Die Haut 2 wird von einem Katheter 4 durchdrungen, was insbesondere aus Figur 2 ersichtlich ist. Der Katheter 4 durchdringt die Haut 2 an einer Katheter-Austrittsstelle 5. Diese wird zur Vermeidung einer Traumatisierung der dort befindlichen Hautzellen durch die Vorrichtung 1 stabilisiert.

Die Vorrichtung 1 besteht aus einer im Grundriß in etwa rechteckigen Trägerschicht 10. Die Trägerschicht 10 ist unterseitig mit einer auf der Haut 2 haftenden Klebeschicht 12 belegt, so daß jeder Laie die Vorrichtung 1 auf der Haut 2 festlegen kann. Vorzugsweise wird hierzu ein hautfreundlicher Klebstoff eingesetzt. Die Trägerschicht 10 bildet zusammen mit der Klebeschicht 12 ein Pflaster. Vorzugsweise besteht die Trägerschicht 10 aus einem lockeren, textilartigen Material, so daß ein hinreichender Luft- und Feuchtigkeitsaustausch der Haut 2 gewährleistet ist.

Damit die Vorrichtung 1 die Haut 2 in der Nähe der Austrittsstelle 5 des Katheters 4 stabilisieren kann, ist die Trägerschicht 10 mit einem zug- und schubfesten Material 13 belegt. Das Material 13 ist dabei mit der Trägerschicht 10 entweder verklebt oder verschweißt. Dies gewährleistet, daß von der Haut 2 gegen den Katheter 4 ausgeübte Zug- oder Schubkräfte vom stabilisierenden Material 13 abgefangen werden. Die Zellen in der Nähe der Austrittsstelle 5 des Katheters 4 werden daher nicht mehr traumatisiert, was die Infektionsgefahr der Austrittsstelle 5 erheblich mindert. Damit die Haut 2 auch im Bereich des zugfesten Materials 13 Luft und Feuchtigkeit austauschen kann, ist das zugfeste Material 13 gitterartig aufgebaut.

Die Trägerschicht 10 steht über das zugfeste Material 13 randseitig über und zeigt einen breiten, weichen Randstreifen 14. Da die Trägerschicht 10 zug- und biegeelastisch ausgebildet ist, kann sich der Randstreifen 14 besonders gut an die Oberfläche der Haut 2 anpassen und geht daher eine günstige Verbindung mit der Haut 2 ein. Dies verhindert ein Abheben der Vorrichtung 1 von der Haut 2 insbesondere, wenn die Vorrichtung 1 im Bauchbereich des Patienten vorgesehen ist.

Direkt an der Austrittsstelle 5 des Katheters 4 besitzt die Trägerschicht 10 zusammen mit dem zugfesten Material 13 eine Aussparung 15, durch die der Katheter 4 hindurchgeführt ist. Um den Katheter 4 leicht in die Aussparung 15 einbringen zu können, ist zwischen der Aussparung 15 und dem äußeren Rand 16 der Trägerschicht 10 ein Schnitt 17 vorgesehen, der sowohl die Trägerschicht 10 als auch das zugfeste Material 13 durchsetzt. Im Bereich der Aussparung 15 ist am zugfesten Material 13 eine Wulst 18 vorgesehen, die den Katheter 4 um wenig mehr als 180° umfaßt. Diese Wulst ist innenseitig an die Form des Katheters 4 angepaßt und fixiert diesen in seiner Lage. Hierdurch wird verhindert, daß der Katheter 4 gegenüber der Vorrichtung 1 bewegt wird, was zu einer Traumatisierung der Zellen im Bereich der Katheter-Austrittsstelle 5 führen könnte.

Um den Katheter 4 sicher zu halten, ist an der Oberfläche des zugfesten Materials 13 ein Halteclip 19 vorgesehen, der den Katheter 4 um mehr als 180° umfaßt. Dieser Halteclip 19 ergreift den Katheter 4 und fixiert diesen auch in axialer Richtung. Dadurch wird verhindert, daß der Katheter 4 herausgezogen wird, wenn sich sein Ende beispielsweise in der Kleidung verfängt.

Figur 4 zeigt eine stark vergrößerte Darstellung eines Ausschnitts IV aus Figur 2. Im unteren Bereich ist die Trägerschicht 10 mit der unterseitig angebrachten Klebeschicht 12 dargestellt. Darüber befindet sich das zugfeste Material 13, dessen gitterartiger Aufbau gut zu erkennen ist. Das zugfeste Material 13 wird von längs- und querlaufenden Bändern bzw. Streifen 20 gebildet, die aus Kunststoff bestehen. Diese Bänder bzw. Streifen 20 sind mit Fasern 21 armiert, die beispielsweise aus Kohlenstoff, Glas oder Polyamid bestehen können. Von der Haut 2 hervorgerufene Druck- bzw. Zugkräfte werden über die Klebeschicht 12 auf die Trägerschicht 10 übertragen. Das zugfeste Material 13 ist über eine Klebeschicht 22 mit der Trägerschicht 10 vorzugsweise lösbar verbunden, so daß von der Haut 2 ausgehende Zugkräfte vom zugfesten Material 13 abgefangen werden. Dabei wirken Zug- und Druckkräfte in Längsrichtung der Fasern 21, so daß deren hohe Zugfestigkeit nur sehr geringe Deformationen des Materials 13 unter Zugbelastung zuläßt. Wird dagegen die unter der Vorrichtung 1 liegende Haut 2 beispielsweise durch Anspannen eines Muskels gekrümmt, so wirkt auf das zugfeste Material 13 eine Biegekraft, der die Fasern 21 praktisch keinen Widerstand entgegensetzen. Das Material 13 gibt daher dieser Biegekraft mit geringem Widerstand nach, so daß die Trägerschicht 10 stets auf der Haut 2 haften bleibt.

Figur 5 zeigt eine Darstellung entsprechend der Figur 4 mit einer alternativen Ausbildung des zugfesten Materials 13. Das zugfeste Material 13 besteht aus einer Folie 23, in der mehrere Durchbrechungen 24 vorgesehen sind. Diese Durchbrechungen 24 sorgen für den notwendigen Luft- bzw. Feuchtigkeitsaustausch der Haut 2. Die Folie 23 ist einstückig an die Trägerschicht 10 angeformt, so daß beide in einem Arbeitsschritt hergestellt werden können. Folie 23 und Trägerschicht 10 bestehen aus Kunststoff, vorzugsweise aus Polyethylen. Die dünne Trägerschicht 10 besitzt dabei eine hinreichend große Zugelastizität und paßt sich vor allem gut jeder Hautfaltung an. Demgegenüber ist die relativ dick ausgebildete Folie 23 zug- und stauchfest. Entsprechend Figur 4 könnte auch die Folie 23 mit Fasern armiert sein.

Figur 6 zeigt eine alternative Ausführungsform der Vorrichtung 1 gemäß Figur 1 mit zwei Halteclips 19, 30 für den Katheter 4. Der Halteclip 19 fixiert den Katheter 4 in unmittelbarer Nähe der Austrittsstelle 5. Der Katheter 4 ist an der Oberfläche des zugfesten Materials 13 anliegend aufgewickelt, wobei sein Ende 31 durch den Halteclip 30 fixiert ist. Die Katheter-Austrittsstelle 5 ist samt dem Katheter 4 von einem Pflaster oder einer Abdeckfolie 32 abgedeckt, so daß Schmutz am Eindringen in die Katheter-Austrittsstelle 5 gehindert ist. In dieser Lage des Katheters 4 kann die Person ungehindert duschen, ohne die Katheter-Austrittsstelle 5 durch eindringende Nässe zu gefährden.

## Patentansprüche

1. Vorrichtung zur Stabilisierung der Haut (2) eines Menschen oder Tieres in der Umgebung einer Austrittsstelle (5) eines Katheters (4), wobei die Vorrichtung (1) von einer dünnen, luftdurchlässigen, biegeelastischen Trägerschicht (10) gebildet ist, die eine Aussparung (15) aufweist, welche die Austrittsstelle (5) mindestens teilweise umfassen kann, wobei die Vorrichtung (1) einseitig mit einer Klebeschicht (12) belegt ist, **dadurch gekennzeichnet,** daß die Trägerschicht (10) mit einem zug- und stauchfesten, jedoch biegsamen und luftdurchlässigen Material (13) verstärkt ist.

2. Vorrichtung nach Anspruch 1, **dadurch gekennzeichnet,** daß das verstärkende Material (13) einstückig an der Trägerschicht (10) angeformt ist und durch eine relativ zur Trägerschicht (10) größere Dicke zug- und stauchfest ausgebildet ist.

3. Vorrichtung nach Anspruch 1 oder 2, **dadurch gekennzeichnet,** daß das zug- und stauchfeste Material (13) von gitterartig zusammengesetzten Fäden, Streifen oder Bändern (20) oder von einer mit Durchbrechungen (24) versehene Folie (23) gebildet ist.

4. Vorrichtung nach mindestens einem der Ansprüche 1 bis 3, **dadurch gekennzeichnet,** daß das zug- und stauchfeste Material (13) Kunststoff, vorzugsweise Polyethylen, Polyurethan oder Polyvinylchlorid ist.

5. Vorrichtung nach mindestens einem der Ansprüche 1 bis 4, **dadurch gekennzeichnet,** daß das zug- und stauchfeste Material (13) mit Fasern (21), vorzugsweise aus Kohlenstoff, Glas oder Polyamid armiert ist.

6. Vorrichtung nach mindestens einem der Ansprüche 1 bis 5, **dadurch gekennzeichnet,** daß an der verstärkt ausgebildeten Trägerschicht (13) außenseitig ein zugelastischer, dünner Randstreifen (14) angeformt ist, der mit einer Klebeschicht (12) versehen ist.

7. Vorrichtung nach mindestens einem der Ansprüche 1 bis 6, **dadurch gekennzeichnet,** daß die Aussparung (15) eine in der Trägerschicht (10) vorgesehene Durchbrechung ist, wobei die Trägerschicht (10) vorzugsweise von einem vom äußeren Rand (16) bis zur Aussparung (15) verlaufenden Schnitt (17) durchsetzt ist.

8. Vorrichtung nach mindestens einem der Ansprüche 1 bis 7, **dadurch gekennzeichnet,** daß die verstärkt ausgebildete Trägerschicht (13) am Rand der Aussparung (15) zumindest über einen Teilbereich eine Wulst (18) aufweist, die den Katheter (4) vorzugsweise um mehr als 180° umfaßt und fixiert.

9. Vorrichtung nach mindestens einem der Ansprüche 1 bis 8, **dadurch gekennzeichnet,** daß am zugfesten Material (13) mindestens ein Halteclip (19) für den Katheter (4) vorgesehen ist.

10. Vorrichtung nach mindestens einem der Ansprüche 1 bis 9, **dadurch gekennzeichnet,** daß die Aussparung (15) zusammen mit dem Katheter (4) abgedeckt ist.

## Claims

1. Device for stabilizing human or animal skin (2) in the area surrounding an exit point (5) of a catheter (4), the device (1) being formed by a thin, air-permeable, flexurally elastic support layer (10) having an opening (15) which can at least partially enclose the exit point (5), the device (1) being coated on one side with an adhesive layer (12), characterized in that the support layer (10) is reinforced with a material (13) which is tension-resistant and compression-resistant and yet flexible and air-permeable.

2. Device according to Claim 1, characterized in that the reinforcing material (13) is formed integrally on the support layer (10) and is made tension-resistant and compression-resistant by having a greater thickness relative to the support layer (10).

3. Device according to Claim 1 or 2, characterized in that the tension-resistant and compression-resistant material (13) is made up of filaments, strips or bands (20) combined in a grid formation, or of a foil (23) provided with through-openings (24).

4. Device according to at least one of Claims 1 to 3, characterized in that the tension-resistant and compression-resistant material (13) is plastic, preferably polyethylene, polyurethane or polyvinyl chloride.

5. Device according to at least one of Claims 1 to 4, characterized in that the tension-resistant and .compression-resistant material (13) is reinforced with fibres (21), preferably of carbon, glass or polyamide.

6. Device according to at least one of Claims 1 to 5, characterized in that a stretch-elastic thin edge strip (14) is formed integrally on the outside of the reinforced support layer (13) and is provided with an adhesive layer (12).

7. Device according to at least one of Claims 1 to 6, characterized in that the opening (15) is a throughopening provided in the support layer (10), the support layer (10) preferably having an incision (17) extending from the outer edge (16) to the opening (15).

8. Device according to at least one of Claims 1 to 7, characterized in that the reinforced support layer (13) has at the edge of the opening (15), over at least part of its area, a bead (18) which encloses the catheter (4) preferably by more than 180° and fixes it.

9. Device according to at least one of Claims 1 to 8, characterized in that at least one holding clip (19) for the catheter (4) is provided on the tension-resistant material (13).

10. Device according to at least one of Claims 1 to 9, characterized in that the opening (15) is covered together with the catheter (4).

## Revendications

1. Dispositif pour la stabilisation de la peau (2) d'un être humain ou d'un animal à proximité du point de sortie (5) d'un cathéter (4), le dispositif (1) étant formé d'une couche de support (10) mince, élastique en flexion et perméable à l'air, qui présente un évidement (15) qui peut entourer au moins partiellement le point de sortie (5), le dispositif (1) étant revêtu sur une face d'une couche de colle (12), caractérisé en ce que la couche de support (10) est renforcée avec une matière (13) résistante à la traction et à l'écrasement mais flexible et perméable à l'air.

2. Dispositif selon la revendication 1, caractérisé en ce que la matière de renfort (13) est formée d'une seule pièce sur la couche de support (10) et est réalisée d'une manière résistante à la traction et à l'écrasement grâce à une épaisseur relativement élevée par rapport à la couche de support (10).

3. Dispositif selon la revendication 1 ou 2, caractérisé en ce que la matière (13) résistante à la traction et à l'écrasement est formée de fils, de bandes ou de rubans (20) réunis à la manière d'un réseau, ou d'une feuille (23) pourvue d'ouvertures (24).

4. Dispositif selon au moins l'une des revendications 1 à 3, caractérisé en ce que la matière (13) résistante à la traction et à l'écrasement est une matière plastique, de préférence du polyéthylène, du polyuréthanne ou du chlorure de polyvinyle.

5. Dispositif selon au moins l'une des revendications 1 à 4, caractérisé en ce que la matière (13) résistante à la traction et à l'écrasement est armée avec des fibres (21), de préférence de carbone, de verre ou de polyamide.

6. Dispositif selon au moins l'une des revendications 1 à 5, caractérisé en ce qu'une bande latérale (14) mince et élastique en traction, qui est pourvue d'une couche de colle (12), est formée le long de la couche de support renforcée (13).

7. Dispositif selon au moins l'une des revendications 1 à 6, caractérisé en ce que l'évidement (15) est une ouverture prévue dans la couche de support (10), la couche de support (10) étant traversée, de préférence, par une incision (17) allant du bord extérieur (16) jusqu'à l'évidement (15).

8. Dispositif selon au moins l'une des revendications 1 à 7, caractérisé en ce que la couche de support (13) renforcée présente, sur le bord de l'évidement (15), au moins sur une zone partielle, un renflement (18) qui de préférence entoure sur plus de 180° et fixe le cathéter (4).

9. Dispositif selon au moins l'une des revendications 1 à 8, caractérisé en ce qu'au moins une pince de maintien (19) pour le cathéter (4) est prévu sur la matière résistante à la traction (13).

10. Dispositif selon au moins l'une des revendications 1 à 9, caractérisé en ce que l'évidement (15) est recouvert conjointement avec le cathéter (4).
